Europäisches Patentamt

European Patent Office

Office européen des brevets

(11) Numéro de publication:

**0 082 041**
**A1**

## DEMANDE DE BREVET EUROPEEN

(21) Numéro de dépôt: 82402173.7

(22) Date de dépôt: 29.11.82

(51) Int. Cl.³: **A 61 M 16/00**

(30) Priorité: 11.12.81 FR 8123193

(43) Date de publication de la demande: 22.06.83
Bulletin 83/25

(84) Etats contractants désignés: **AT BE CH DE FR GB IT LI LU NL SE**

(71) Demandeur: **SYNTHELABO, 58 rue de la Glacière, F-75013 Paris (FR)**

(72) Inventeur: **Chopin, Claude, Hameau du Drumetz Route des Cailles, F-59710 Attiches (FR)**
Inventeur: **Caillot, Luc, 39, rue de l'Amiral Mouchez, F-75013 Paris (FR)**

(74) Mandataire: **Pinguet, André, CAPRI 28 bis, avenue Mozart, F-75016 Paris (FR)**

(54) Procédé et dispositif pour commander la respiration artificielle.

(57) La mise à l'arrêt ou en marche du respirateur (1) est commandée par un module (11) recevant d'un capnigraphe (8) un signal électrique représentatif de la teneur en $CO_2$ du gaz traversant une cellule (6) d'analyse du $CO_2$ fixée à la sortie de la sonde d'intubation, de trachéotomie d'un patient en respiration assistée. C'est la teneur en $CO_2$ qui détermine la marche ou l'arrêt de l'assistance par le respirateur.

EP 0 082 041 A1

Procédé et dispositif pour commander la respiration artificielle.

La présente invention a pour objet un perfectionnement aux respirateurs ou ventilateurs artificiels et plus particulièrement un nouvel appareillage destiné à faciliter le sevrage des malades soumis à l'assistance ventilatoire et la surveillance des modes de ventilation spontanée (VS-CPAP IMV). Elle vise également un nouveau mode opératoire de commande de la respiration artificielle.

Il existe actuellement des systèmes permettant la ventilation à la demande au cours du sevrage des malades soumis à l'assistance ventilatoire pour un épisode d'insuffisance respiratoire aiguë. Parmi ces systèmes on connait notamment :

- la mise en ventilation spontanée avec utilisation du respirateur qui délivre au malade un mélange gazeux, à concentration en oxygène, température et humidification constantes. La spirométrie peut être surveillée. En cas d'apnée (absence de respiration) le ventilateur reprend la commande après un délai constant. L'adjonction d'une pression positive résiduelle est possible ; elle est dénommée couramment CPAP, d'après l'appellation en anglais : Continuous Positive Airway Pressure.

- l'utilisation du Trigger ou ventilation auto-déclenchée, le malade maitrisant la fréquence de fonctionnement du respirateur.

- la ventilation intermittente à la demande qui permet une assistance ventilatoire partielle à raison de n cycles spontanés pour 1 cycle controlé (2/1, 3/1, 4/1, etc). Ce système peut fonctionner sur un mode synchrone ou non synchrone. Il est désigné couramment IMV, d'après l'appellation en anglais : Intermittent Mandatory Ventilation.

- la ventilation forcée déterminée dans laquelle est définie une spirométrie de base que le malade doit dépasser en ventilation spontanée. Si la spirométrie spontanée du malade est inférieure à une limite que le médecin peut fixer, le ventilateur reprend la commande. Ce système est désigné habituellement par MMV, également d'après l'appellation en anglais : Minute Mandatory Ventilation.

Aucun de ces systèmes n'est pleinement satisfaisant. En effet, aucun d'entre eux ne tient compte de l'efficacité de la ventilation spontanée du malade.

Conformément à la présente invention, la ventilation est asservie à la teneur en gaz carbonique du gaz expiré par le patient. Selon la présente invention, on adjoint à un respirateur artificiel une cellule d'analyse de la teneur en $CO_2$ du gaz expiré, un capnigraphe, appareil qui reçoit de la cellule la teneur en $CO_2$ et la transforme en un signal électrique analogique, et un module de ventilation à la demande asservi à la teneur en $CO_2$, appareil qui reçoit le signal analogique du capnigraphe et qui commande la marche du ventilateur.

La cellule et le capnigraphe sont des appareils connus, disponibles dans le commerce. Le module est nouveau et constitue une caractéristique importante de la présente invention.

Le système selon l'invention, qui comporte ainsi un procédé et un dispositif, tient compte de l'efficacité respiratoire du malade et permet le sevrage dans des conditions de sécurité maximum, pour le minimum d'intervention du personnel soignant.

D'autres caractéristiques de l'invention apparaîtront au cours de la description qui va suivre, donnée à titre d'exemple non limitatif en regard des dessins ci-joints, et qui fera bien comprendre comment l'invention peut être réalisée.

Les dessins montrent :

- figure 1 : un schéma général du dispositif selon l'invention ;
- figure 2 : une représentation d'un signal analogue délivré par capnigraphe ;
- figure 3 : un schéma bloc du module selon l'invention ;
- figure 4 : un schéma complet avec indication des composants du module de la figure 3;
- figure 5 : une représentation de l'évolution de la pression partielle de $CO_2$ dans le sang ($PaCO_2$) au cours d'une journée, avec utilisation du dispositif selon l'invention ; et
- figure 6 : une représentation du signal de sortie du capnigraphe au cours d'une séquence de sevrage progressif avec passage en mode à ventilation intermittente (IMV).

Dans la suite de la description, on décrira l'emploi d'éléments bistables qui peuvent prendre deux états : un état bas, ou zéro et un état haut ou un. On utilisera pour simplifier les expressions zéro et un sans y attribuer un caractère limitatif.

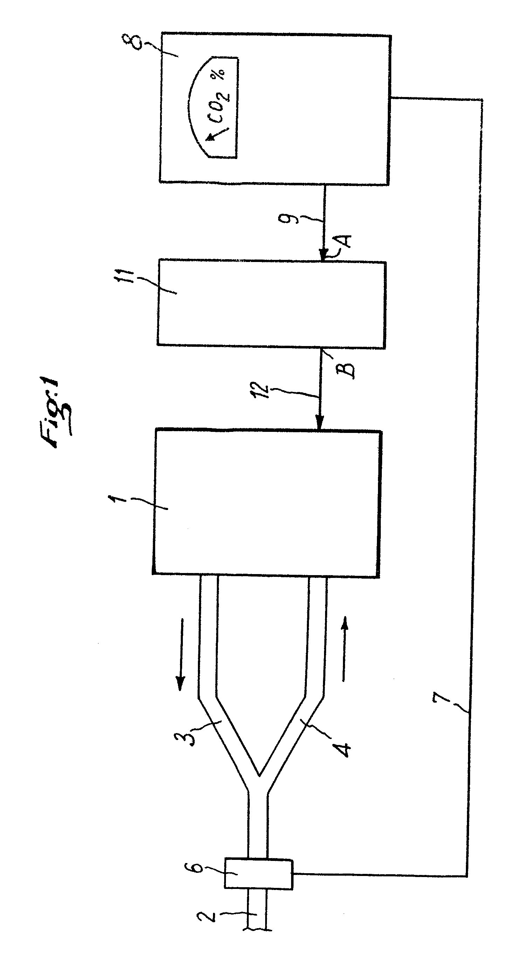

Comme on peut le voir sur la figure 1, un dispositif selon l'invention comporte un ventilateur, ou respirateur artificiel 1, qui peut être d'un type quelconque, branché sur un patient au moyen d'une sonde d'intubation, de trachéotomie, ou moins souvent d'un masque 2, alimenté par un conduit d'inspiration 3 et un conduit d'expiration 4. Sur l'espace mort d'appareillage (partie du circuit balayée alternativement par le gaz inspiré et par le gaz expiré) est placée une cellule 6 d'analyse du $CO_2$, connectée par une ligne 7 à un capnigraphe 8. Celui-ci est connecté par une ligne 9 à l'entrée A du module 11 de ventilation à la demande ($M.V.CO_2$, d'après l'appellation en langue anglaise Mandatory Ventilation), dont la sortie B est connectée par une ligne 12 au ventilateur 1.

L'appareil peut s'adapter à tous les ventilateurs modernes, possédant un mode de fonctionnement spontané. La capnigraphie est recueillie au niveau de l'espace mort d'appareillage (figure 1). La concentration maxima (en fin d'expiration) de $CO_2$ expiré par le malade ($FCO_{2ET}$ ; ET = End Tidal ou fin de cycle) est la valeur constamment surveillée.

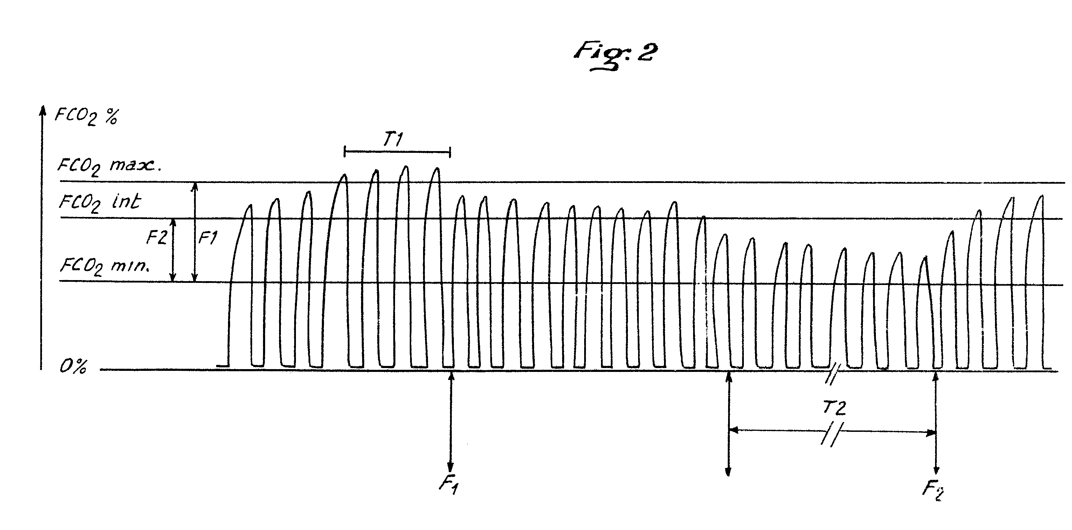

La figure 2 représente le signal analogique de sortie du capnigraphe. Pendant l'inspiration, la concentration en $CO_2$ est nulle. Chaque dent de la courbe représente une période d'expiration : la concentration est maximum en fin d'expiration.

Le médecin détermine deux fourchettes de surveillance ; la première entre un seuil minimum et maximum : c'est la fourchette n° 1 (F1) -figure 2- dite de ventilation spontanée ; la seconde entre le même seuil minimum et un seuil intermédiaire inférieur au seuil maximum, c'est la fourchette n° 2 (F2), dite de ventilation contrôlée.

Le seuil maximum ($FCO_2$max) est calculé par la différence entre $PCO_{2ET}$ (pression partielle du $CO_2$ expiré de fin d'expiration) et la pression partielle de $CO_2$ dans le sang artériel ($PaCO_2$). Ce gradient est une caractéristique du malade au moment du sevrage. (Le sevrage est la période d'enlèvement du ventilateur à partir du moment où le patient a une respiration autonome acceptable). Il nécessite une analyse des gaz du sang artériel pour sa détermination.

$$PCO_{2ET} = FCO_{2ET} \times (PB - PBH_2O) \quad \text{ou} \quad FCO_{2ET} = \frac{PCO_{2ET}}{(PB - PBH_2O)}$$

$$PCO_{2ET} = PaCO_2 - (PaCO_2 - PCO_{2ET})$$

où PB est la pression barométrique et $PBH_2O$ est la pression partielle de vapeur d'eau, $FCO_{2ET}$ la concentration en $CO_2$ du gaz expiré en fin d'expiration, $PCO_{2ET}$ la pression partielle du $CO_2$ du gaz expiré en fin d'expiration, $PaCO_2$ la pression partielle du $CO_2$ dans le sang.

Par exemple, si l'on désire que la $PaCO_2$ du malade ne dépasse pas 45 mmHg, pour un gradient de 12 mmHg :

$PCO_{2ET}$ = 33 mmHg = 45 - 12

$FCO_{2ET}$ = 33/730      pour PB = 760 et $PBH_2O$ = 47 à 37°C

$FCO_{2ET}$ = 4,52%

Le seuil maximum de $FCO_2$max sera fixé à 4,5%.

Le seuil minimum ($FCO_2$min) est toujours supérieur à 1% de $CO_2$. Il permet la reprise de la commande par le ventilateur en cas d'apnée, de défaillance cardio-circulatoire aiguë, d'arrêt cardiaque ou de tachycardie très importante.

Le seuil intermédiaire ($FCO_2$int) est toujours compris entre le $FCO_2$max et $FCO_2$min. Il est nécessaire que, dans le mode de ventilation contrôlée, $FCO_{2ET}$ soit compris entre $FCO_2$min et $FCO_2$int.

A la mise en route du système, le malade est en ventilation contrôlée et les valeurs de $FCO_2$min et de $FCO_2$int sont déterminées ; puis le malade est mis en ventilation spontanée et $FCO_2$max est déterminée.

Tant que le malade présente une ventilation satisfaisante avec

$$FCO_2\text{min} < FCO_{2ET} < FCO_2\text{max}$$

il reste en ventilation spontanée. Dès qu'il dépasse les seuils (en pratique, il dépasse le plus souvent le seuil maximum, $FCO_2$max), l'appareil enclenche automatiquement le mode "ventilation contrôlée", si le dépassement est constant et d'une durée supérieure à une période dont la durée variable peut être fixée par exemple à 15 ou 20 s (temps de temporisation T1). En ventilation contrôlée $FCO_{2ET}$ diminue rapidement et doit se situer à nouveau entre $FCO_2$min et $FCO_2$int.

$$FCO_2\text{min} < FCO_{2ET} < FCO_2\text{int}$$

Si $FCO_{2ET}$ reste effectivement compris entre ces deux seuils pendant une période pouvant être fixée par exemple à 5 mn (temps de temporisation T2), l'appareil enclenche alors le mode de ventilation spontanée. T1 et T2 sont réglables dans leur durée de façon interne à l'appareil. Entre la fin de T1 et la fin de T2 (figure 2) la ventilation est contrôlée, en dehors de cette période, elle est spontanée.

Toute absence de cycle, apnée en inspiration ou en expiration, enclenche automatiquement le mode de ventilation contrôlée après un temps égal à T1.

Toute panne du capnigraphe, toute absence d'alimentation du système enclenche également le mode contrôlé, de même que l'arrêt volontaire de l'appareil, ou par défaut d'alimentation.

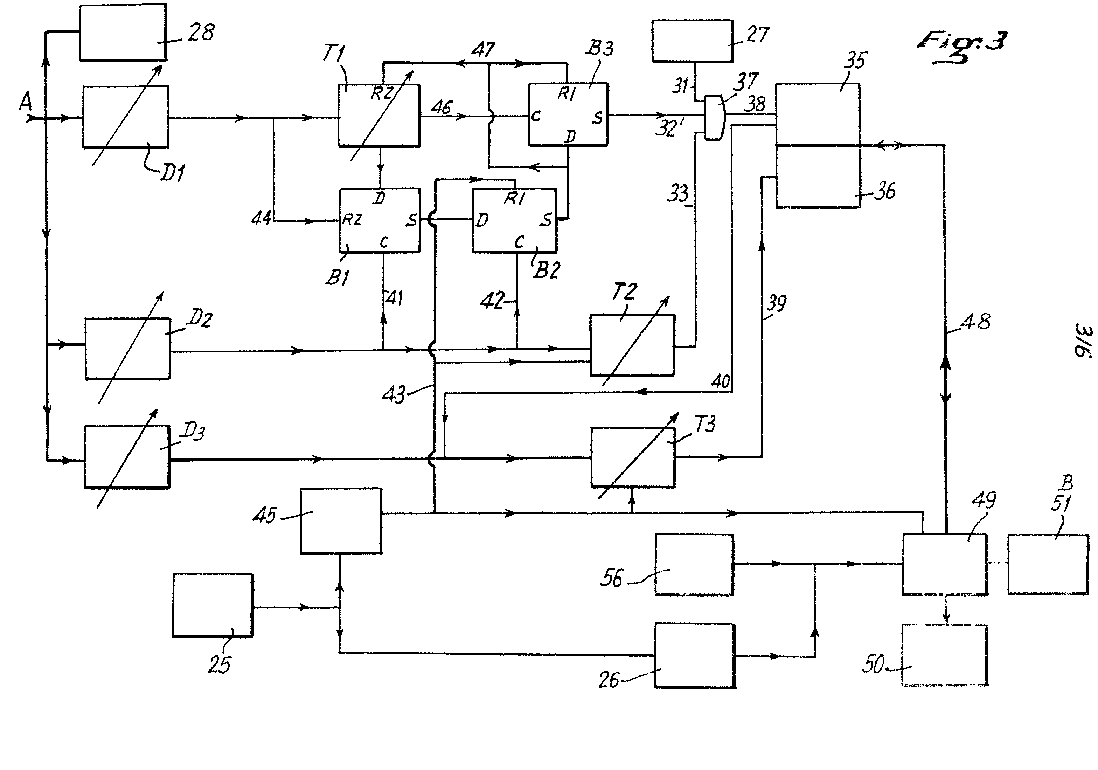

L'appareil dispose d'une commande mise en attente (stand by) 56, figure 3, qui suspend son fonctionnement durant les aspirations et soins du malade. Le mode ventilation contrôlée est alors adopté. L'arrêt de la position "stand by" fait redémarrer le système dans le mode qui était le sien avant la suspension.

La figure 3 représente le schéma de principe du module 11 de la figure 1. Le signal $CO_2$ provenant du capnigraphe 8 est délivré au point A. A titre d'exemple commode, une concentration de 1% de $CO_2$ peut être représentée par une tension de 1 volt. Le module comporte une alimentation 25 avec un détecteur de pannes secteur 26, et une commande manuelle 27. On peut avantageusement prévoir un affichage 28 de la teneur en $CO_2$. Le module comporte trois détecteurs de seuil : un détecteur de seuil maxi D1, un détecteur de seuil mini D2 et un détecteur de seuil intermédiaire D3. Dans une forme de réalisation de l'invention, ces détecteurs peuvent être des bascules qui prennent sur leurs sorties l'état zéro quand la tension à l'entrée est supérieure à la tension choisie, et l'état un, quand la tension d'entrée est inférieure.

0082041

La sortie B du module est branchée sur la connexion 51 au respirateur, reliée à travers la mémoire 49 (mise en mémorisation de l'état du respirateur) à la bascule de commande 35, 36 : 35 mise en marche du respirateur, 36 mise à l'arrêt du respirateur. Seuls les états zéro font changer la position de la bascule 35, 36.

Les détecteurs D1, D2, D3 sont connectés chacun à une temporisation T1, T2, T3.

T1 est connecté, à travers une bascule B3 à l'entrée 32 d'une porte ET 37 dont la sortie 38 est connectée à la mise en marche 35 ;

T2 est connecté à l'entrée 33 de la porte 37 et

T3 est connecté par la ligne 39 à la mise à l'arrêt 36.

La commande manuelle 27 est connectée à l'entrée 31 de la porte 37. Si les trois entrées 31, 32, 33 de la porte 37 sont à l'état un, la sortie 38 est à l'état un, ce qui n'a pas d'action sur la mise en marche. Si une des entrées 31, 32, 33 est à l'état zéro, la sortie 38 est l'état zéro, ce qui déclenche la mise en marche 35 du respirateur. Quand D1 passe à l'état zéro (dépassement de $FCO_2max$) T1 prend l'état un pour (par exemple) 15 s. Quand D2 passe à l'état zéro (dépassement de $FCO_2min$) T2 prend l'état un pour 15 s. Si D2 garde l'état un, c'est-à-dire si $FCO_2min$ n'est pas atteint pendant 15 s, T2 passe à l'état zéro ce qui déclenche la mise en marche (dès qu'une des entrées 31, 32, 33 est à l'état zéro), ce qui déclenche l'alarme sonore 50, à travers la mémoire 49. La mise en marche du respirateur déclenche par la ligne 40 une impulsion qui met T3 à l'état un pour une série de 5 mn (par exemple). L'état zéro de D3 (dépassement de $FCO_2int$) maintient chaque fois T3 à l'état un pour 5 mn. Si pendant 5 mn T3 ne reçoit rien de D3 (pas de dépassement de $FCO_2int$, D3 reste à l'état un) T3 passe à l'état zéro, ce qui commande l'arrêt 36 du respirateur. Deux bascules B1 et B2 sont montées comme représentées. D1 est connectée sur la remise à zéro de B1 par la ligne 44, T1 est connectée à l'entrée D de B1, dont la commande C est connectée par la ligne 41 à la sortie de D2. La sortie S de B1 est connectée à l'entrée D de B2 dont la commande C est connectée par la ligne 42 également à la sortie de D2. La remise à un de B2 est connectée par la ligne 43 à la remise à zéro automatique 45. La sortie S de B2 est connectée à l'entrée D de B3 dont la commande C est connectée par la ligne 46 de T1. La remise à un de B3 est connectée par la ligne 47 à la sortie S de B2, la ligne 47 étant connectée aussi à la remise à zéro de T1. La sortie S de B3 est connectée à l'entrée 32 de la porte 37. Les basculent sont réglées de la façon suivante. Pour B1, un état zéro de D2, par la ligne 41 fait passer en S l'état de D. Pour B2, un état un de D2, par la ligne 42 fait passer en S l'état de D. Pour B3, T1 envoie une impulsion sur la commande C à la fin de la période de temporisation de 15 s de T1. Cette impulsion fait passer à S l'état de D.

Le module fonctionne de la façon suivante.

A la mise en route de l'appareil, le système de Raz automatique envoie une impulsion sur :
- la remise à un de la bascule B2 dont la sortie prend l'état un, cette sortie commande la remise à un de la bascule B3 et la remise à zéro du temporisateur T1, la bascule 3 prend donc sur sa sortie l'état un, T1 l'état zéro.
- l'entrée du temporisateur T2 qui prend l'état un sur sa sortie pour 15s.
- la remise à zéro du temporisateur T3 qui prend sur sa sortie l'état zéro.
- la remise à zéro de la mémoire du stand by 46.
Donc à la mise en route de l'appareil on a :
B3 = 1
T2 = 1
T3 = 0

Comme seuls les états zéro font changer de position la bascule de mise en marche ou de mise à l'arrêt du respirateur, au départ on a arrêt du respirateur (T3 = 0).

0082041

Si au bout de 15s T2 n'a pas reçu d'impulsion sur son entrée venant de D2, celui-ci passe à l'état zéro et le respirateur se met en fonctionnement; dès l'instant où celui-ci se met en marche , T3 reçoit une impulsion et temporise pour un temps réglé par exemple à 5 mn.

Si au bout de 5 mn celui-ci n'a plus reçu d'impulsion venant de D3 ou d'un second ordre éventuel de mise en marche du respirateur, la sortie de T3 passe alors à zéro et on a arrêt du respirateur.

La détection maxi s'opère comme suit. Au moment où $FCO_2max$ est dépassé, D1 prend l'état zéro, T1 passe à un pour 15s et B1 est mise à zéro par la ligne 44. A la fin de l'expiration commence la période d'inspiration suivante avec $FCO_2 = 0$ donc les trois détecteurs prennent l'état un. L'état un de D2 est appliqué par les lignes 41 et 42 sur les entrées C de B1 et B2. Cela n'a aucun effet sur B1 qui n'est sensible qu'à un état zéro, mais B2 est sensible à l'état un. Comme la sortie S de B1 est à l'état zéro, qui est appliqué à l'entrée D de B2, cet état est transmis à la sortie S de B2 par le passage à l'état un de D2. A la remontée due à l'expiration suivante, D2 reprend l'état zéro ce qui fait prendre à la sortie S de B1 l'état de T1, c'est-à-dire un.

Deux cas peuvent se présenter :
1) on refranchit le seuil D1 : B1 est remis à zéro, puis on repasse sous le seuil D2, la sortie S de B2 prend l'état de B1, c'est-à-dire zéro, qu'elle avait déjà. Cet état zéro est appliqué à l'entrée D de B3 mais n'est pas transmis à la sortie qui reste à l'état un. Donc chaque fois que $FCO_2$ franchit seuil maxi, seuil mini, seuil maxi ... etc l'opération se répète pendant tout le temps de la temporisation de T1. T2 est à l'état un, la commande manuelle aussi en l'absence d'actionnement, les trois entrées 31, 32, 33 de la bascule 37 étant à l'état un, celle-ci ne déclenche pas la mise en marche 35 du respirateur. Au moment où T1 revient à zéro, il envoie une impulsion sur la commande C de B3, ce qui fait passer la sortie S de B3 à l'état de D, c'est-à-dire l'état S de B2 qui reste à zéro. Le passage de la sortie S de B3 à l'état zéro commande la mise en marche du respirateur.

2) on ne refranchit pas le seuil D1 au cours de la temporisation de T1 mais on franchit le seuil D2. B1 n'est pas remise à zéro ; B2 prend alors l'état un quand D2 prend l'état un ce qui, par la ligne 47, met la sortie de B3 à l'état un et remet à zéro T1. Rien ne se passe et le respirateur reste à l'arrêt.

En résumé, si D1 est franchi entre un franchissement vers le haut et un franchissement vers le bas de D2, B2 reste à l'état zéro et le respirateur se met en marche au bout du temps T1. Mais si D2 est franchi vers le haut, puis vers le bas, sans que D1 ait été dépassé, B2 se met à l'état un lors du passage de D2 vers le bas. Si B2 est à l'état un à la fin de T1, il n'y a pas mise en route du respirateur.

En cas de coupure secteur ou de mise en stand by par la commande 56, l'état du respirateur est mis en mémoire en 49 et est restitué lorsque le secteur revient ou lorsque l'on relâche le bouton stand by.

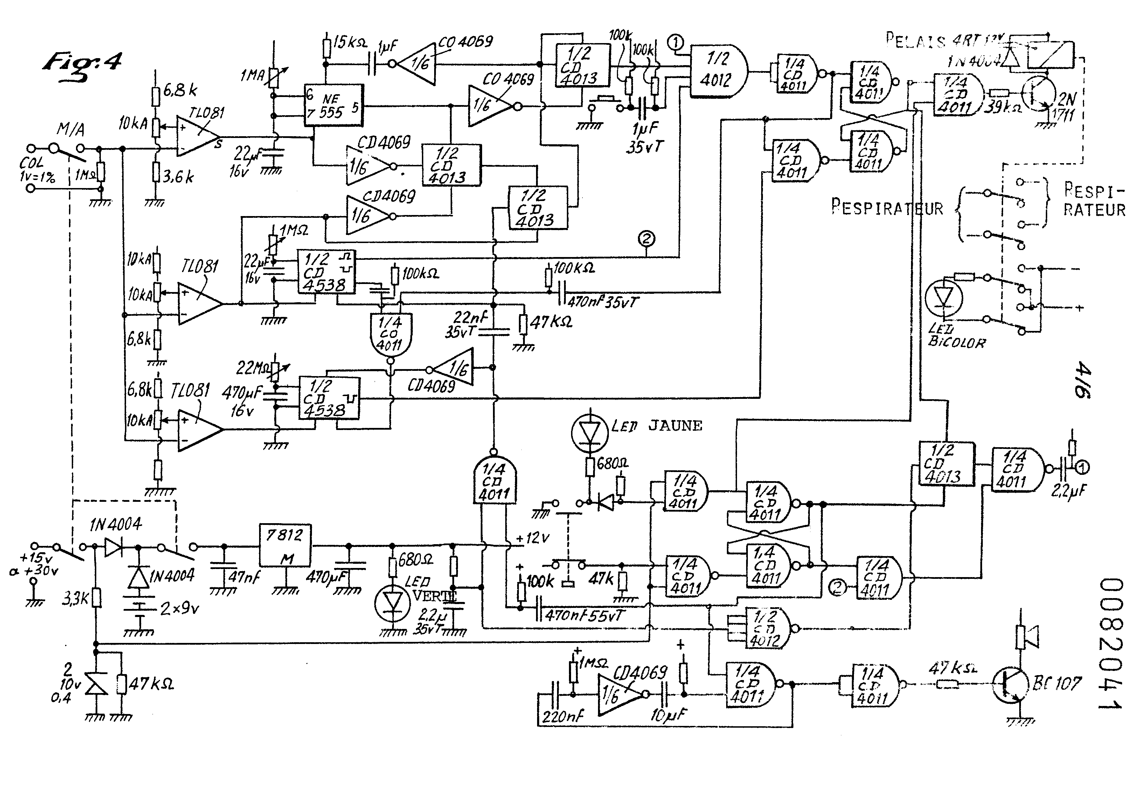

La figure 4 représente un exemple de montage pratique avec les signes conventionnels et les valeurs des composants. L'homme de l'art peut ainsi réaliser complètement le dispositif de l'invention. Il est évident que des variantes ou des équivalents sont possibles, tant dans le détail du montage que du choix des composants ou de leurs valeurs.

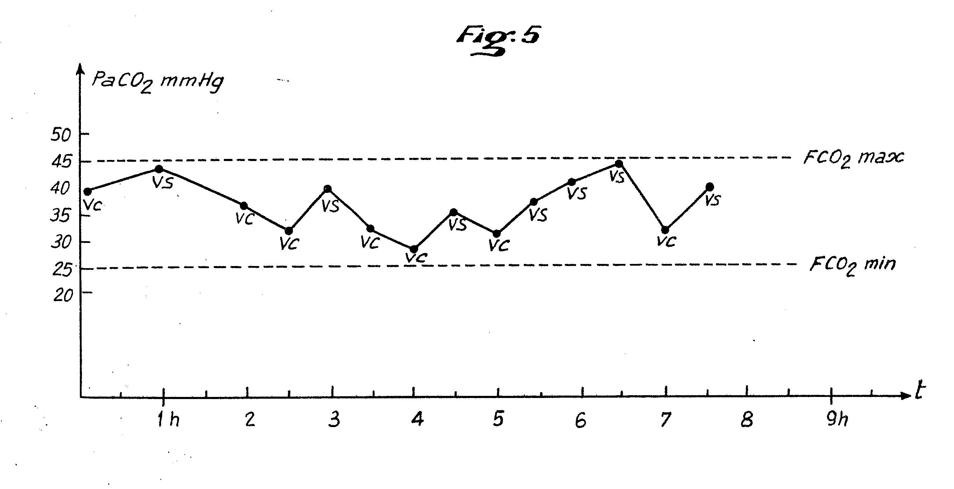

La figure 5 représente l'évolution de la teneur en $CO_2$ du gaz expiré au cours d'une journée. VC signifie ventilation contrôlée et VS ventilation spontanée. On voit que l'appareil a l'avantage de maintenir une $PaCO_2$ constante au

cours de la journée.

La figure 2 représente une séquence de ventilation spontanée, jusqu'à la fin    F1 de T1, ventilation contrôlée jusqu'à la fin    F2 de T2, puis ventilation spontanée à nouveau.

L'utilisation d'un microprocesseur permettra une détection plus précoce de la valeur de $FCO_{2ET}$ et un moyennage sur trois cycles, de fixer plus précisément les valeurs des différents seuils, de fixer des durées variables suivant les malades pour T1 et T2, et surtout d'envisager entre le mode de ventilation contrôlée et le mode spontané, un passage en ventilation intermittente à la demande (IMV) progressivement dégradé.

Le passage ventilation spontanée ⟶ ventilation contrôlée s'effectuera donc comme précédemment, mais le passage ventilation contrôlée ⟶ ventilation spontanée s'effectuera plus progressivement permettant une meilleure adaptation du malade à la ventilation spontanée.

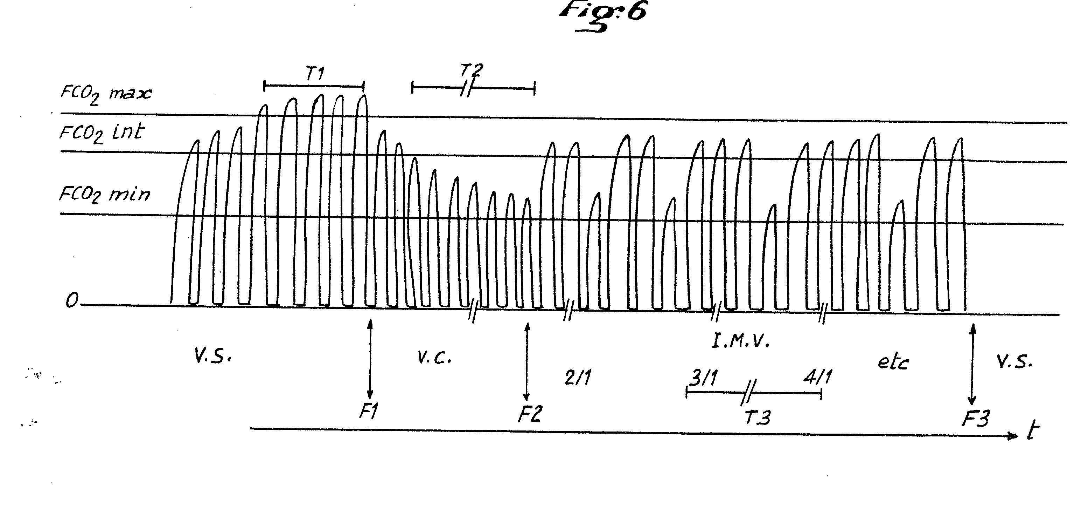

La figure 6 représente une période de sevrage progressif avec passage en mode IMV : ventilation forcée intermittente. On a une ventilation spontanée jusqu'à la fin F1 de T1, une ventilation contrôlée jusqu'à la fin F2 de T2 puis une ventilation IMV jusqu'à F3 et ensuite une ventilation spontanée.

Tout dépassement en mode IMV pendant une durée supérieure à T1 de $FCO_2max$ enclenche le mode contrôlé et la procédure recommence.

Le temps T3 = durée de chacun des types IMV (2/1, 3/1, 4/1, etc) peut être fixe, de l'ordre de 3 mn, ou variable, réglable par le médecin pour chaque malade.

L'utilisation d'un mircoprocesseur pourra également permettre de tenir compte de la fréquence ventilatoire dans le mode spontané ; le dépassement d'un seuil maxima et minima entraînant la mise en marche du respirateur dans le mode contrôlé. Cette prise en compte de la fréquence ventilatoire est une amélioration possible du système.

Revendications

1. Procédé de commande de la respiration artificielle d'un patient placé sous l'assistance d'un respirateur artificiel caractérisé en ce que la mise en marche et l'arrêt du respirateur sont commandés en fonction de la teneur en $CO_2$ en fin d'expiration $FCO_{2ET}$ du gaz expiré par le patient.

2. Procédé selon la revendication 1, caractérisé en ce que l'on détermine un seuil maximum $FCO_2max$ et un seuil minimum $FCO_2min$ de la teneur en $CO_2$ du gaz expiré par le patient et que tant que le patient présente une ventilation spontanée avec $FCO_2min < FCO_{2ET} < FCO_2max$ il reste en ventilation spontanée, et dès qu'il dépasse les seuils, de façon constante pendant une durée supérieure à une durée choisie arbitrairement, on met le patient en ventilation contrôlée.

3. Procédé selon la revendication 2, caractérisé en ce que l'on détermine un troisième seuil $FCO_2int$ intermédiaire entre les deux premiers et que l'on arrête la ventilation contrôlée si l'on a
$$FCO_2min < FCO_{2ET} < FCO_2int$$
pendant une période choisie de l'ordre de 5 mn.

4. Dispositif pour la mise en oeuvre du procédé selon une des revendications 1 à 3, caractérisé en ce qu'il comprend un respirateur artificiel (1) avec une conduite (3) d'inspiration et une conduite (4) d'expiration connectées toutes deux à une sonde d'intubation, de trachéotomie, ou à un masque (2), une cellule (6) d'analyse de la teneur en $CO_2$ du gaz qui la traverse placée sur une conduite commune à l'aspiration et à l'expiration, un capnigraphe (8) connecté à la cellule et transformant les signaux reçus de celle-ci pour délivrer des signaux transformés à un module de commande, et un module (11) de commande recevant les signaux du capnigraphe pour commander la mise en marche ou la mise à l'arrêt du respirateur, le module étant organisé pour commander la mise en marche ou la mise à l'arrêt du respirateur en fonction de la teneur en $CO_2$ en fin d'expiration du gaz expiré.

5. Dispositif selon la revendication 4, caractérisé en ce que le capnigraphe (8) délivre au module une tension électrique proportionnelle à chaque instant à la teneur en $CO_2$ du gaz aspiré ou expiré, que le module (11) comporte trois détecteurs D1, D2, D3 disposés en parallèles et auxquels est appliquée la sortie du capnigraphe, les détecteurs étant réglables en tension de façon à prendre un état ou un autre selon que la tension appliquée est supérieure ou inférieure à la tension de réglage, et un circuit logique tel que, si l'on désigne par $FCO_{2ET}$ la tension maxima délivrée par le capnigraphe à chaque expiration , pour

$$D2 < FCO_{2ET} < D1$$

le circuit logique ne commande pas la mise en marche du ventilateur mais la commande, si $FCO_{2ET}$ sort des limites D2, D1 de façon constante pendant une durée choisie de l'ordre de 15s, et commande la mise à l'arrêt du ventilateur si la condition $D2 < FCO_{2ET} < D3$ est satisfaite pendant une période de temps choisie, de l'ordre de quelques minutes : 3 à 5.

6. Dispositif selon la revendication 5, caractérisé en ce que la mise en marche ou à l'arrêt du ventilateur est commandée par une bascule (35,36) sensible à un état zéro sur une de ses entrées, que les détecteurs D1, D2, D3 sont des bistables prenant l'état zéro quand la tension appliquée est supérieure à la tension de réglage, et un état un (haut) quand la tension appliquée est inférieure à la tension de réglage, chaque détecteur D1, D2, D3 étant connecté directement à une temporisation associée respectivement T1, T2, T3.

7. Dispositif selon la revendication 6, caractérisé en ce que la temporisation T1 est branchée sur la commande (35,

0082041

36) du respirateur à travers une bascule B3 dont le niveau est commandé par deux bascules B1, B2 en série, le niveau de B1 étant commandé par D1 et D2 et le niveau de B2 étant commandé par B1 et D2.

BAD ORIGINAL

Fig.1

Fig. 2

Fig.3

0082041

3/6

Fig.4

Fig.5

5/6

0082041

Fig: 6

0082041

Office européen
des brevets

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | A 61 M 16/00 |
| A | MEDICAL & BIOLOGICAL ENGINEERING, vol. 13, no. 6, novembre 1975, pages 846-854, Stevenage, Herts (GB); Y.MITAMURA et al.: "A dual control system for assisting respiration". *Figures 3,4; page 848, colonne de droite, dernier alinéa - page 849, colonne de gauche, premier alinéa* | 1 | |
| | --- | | |
| A | EP-A-0 022 144 (DRÄGERWERK AG) *Figure 1; page 4, lignes 14-29; page 6, lignes 17-23* | 1 | |
| | --- | | |
| A | GB-A- 798 561 (A.ST.JACQUES LEE) *Figure 1; page 6, ligne 21 - page 7, ligne 17* | 1 | DOMAINES TECHNIQUES RECHERCHES (Int. Cl. ³) |
| | --- | | A 61 M |
| A | DE-B-2 424 025 (ORIGINAL HANAU GmbH) *Figures 1-4; colonne 11, linges 36-48; colonne 14, lignes 3-19* | 1 | G 01 N |
| | --- | | |
| A,P | GB-A-2 079 984 (ENGSTRÖM MEDICAL AB) *Page 2, lignes 64-89* | 1 | |
| | ----- | | |
| | Le présent rapport de recherche a été établi pour toutes les revendications | | |

| Lieu de la recherche LA HAYE | Date d'achèvement de la recherche 25-01-1983 | Examinateur VEREECKE A. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form I503 03 82